# EUROPÄISCHE PATENTANMELDUNG

(11) **EP 1 295 860 A2**
(43) Veröffentlichungstag der Anmeldung: **26.03.2003**
(21) Anmeldenummer: 02019871.9
(22) Anmeldetag: 10.09.2002
(51) Int. Cl.: C07C 51/09, C07C 57/10

(54) **Verfahren zur Herstellung von Sorbinsäure durch thermische Spaltung**

(30) Priorität: 21.09.2001 DE 10146661
(71) Anmelder: Nutrinova Nutrition Specialties & Food Ingredients GmbH, 65929 Frankfurt am Main (DE)
(72) Erfinder: Decker, Daniel Dr., 65843 Sulzbach (DE); Roscher, Günter Dr., 65779 Kelkheim (DE); Mollenkopf, Christoph Dr., 65929 Frankfurt (DE); Weiss, Erwin Dr., 65719 Hofheim (DE); Purps, Stefan, 61462 Königstein (DE)
(74) Vertreter: Schweitzer, Klaus, Dr.

(57) **Zusammenfassung**

Die Erfindung betrifft ein Verfahren zur Herstellung von Sorbinsäure durch thermische Spaltung des aus Crotonaldehyd und Keten hergestellten Polyesters in einem Lösungsmittel, wobei man die Spaltung des Polyesters in Gegenwart von 20 bis 100 Gew.-% eines sekundären oder tertiären aliphatischen, alicyclischen Stickstoff und/oder Sauerstoff enthaltenden oder aliphatisch-aromatisch substituierten Amins als Katalysator durchführt.

## Beschreibung

Die Erfindung betrifft ein Verfahren zur Herstellung von Sorbinsäure. Zur Herstellung von Sorbinsäure sind verschiedene Verfahren bekannt. Ein besonders wirtschaftliches Verfahren geht von dem polymeren Reaktionsprodukt Polyester aus, der durch Umsetzung von Crotonaldehyd mit Keten in Gegenwart eines fettsauren Salzes eines zwei- und/oder dreiwertigen Metalls der II. bis VIII. Nebengruppe des Periodensystems als Katalysator hergestellt wird (DE-A 1 042 573). Die Katalysatoren werden im allgemeinen zwischen 0,1 bis 5 %, vorzugsweise zwischen 0,5 bis 2 % der Gewichtsmenge des eingesetzten Crotonaldehyds verwendet. Der so hergestellte Polyester kann auf verschiedenen Wegen in Sorbinsäure umgewandelt werden.

Ein großtechnisch bedeutsames Verfahren besteht zum Beispiel aus der thermischen katalytischen Spaltung des Polyesters, wobei die Spaltung des Polyesters in einem inerten und unter Normaldruck über 150°C siedenden Verdünnungsmittel (DE-A-1 059 899) in Gegenwart eines sekundären oder tertiären aliphatischen Amins als Katalysator bei Temperaturen von 160 bis 220°C unter gleichzeitigem Abdestillieren der Sorbinsäure und des Lösungsmittels durchgeführt wird (DE-A 1 282 645). Die Spaltung des Polyesters erfolgt in Gegenwart von 0,5 bis 10, vorzugsweise von 2 bis 5 Gewichtsprozent des Katalysator-Amins. In der Patentschrift (DE-B 1 282 645) wird weiter behauptet, dass eine noch größere Aminmenge zu keiner höheren Ausbeute führt.

Die Erfindung betrifft nun ein Verfahren zur Herstellung von Sorbinsäure durch thermische Spaltung des aus Crotonaldehyd und Keten hergestellten Polyesters in einem Lösungsmittel, dadurch gekennzeichnet, dass man die Spaltung des Polyesters in Gegenwart von 20 bis 100 Gew.-%, bezogen auf den eingesetzten Polyester, eines sekundären oder tertiären aliphatischen, alicyclischen Stickstoff und bzw. oder Sauerstoff enthaltenden oder aliphatisch-aromatisch substituierten, unter Normaldruck oberhalb 100°C, vorzugsweise oberhalb 150°C, siedenden Amins als Katalysator durchführt.

Überraschenderweise wurde gefunden, dass bei der wie in DE-A 1 282 645 beschriebenen thermischen Spaltung des Polyesters, anders als dort beschrieben, ein sprunghafter Anstieg der Ausbeute an Sorbinsäure gefunden wird, wenn die Konzentration des Katalysatoramins mindest doppelt so hoch wie die in DE-A-1 282 645 angegebene Konzentrationsobergrenze gewählt wird. Wie in DE-A-1 282 645 beschrieben, führt eine geringe weitere Anhebung der Aminmenge über die dort genannte obere Katalysatormenge zu keiner signifikanten Auswirkung auf die Sorbinsäureausbeute. Erst die starke Erhöhung der Aminkonzentration führt zu einer signifikanten Ausbeutesteigerung.

Vorteilhaft führt man die Spaltung des Polyesters in einem inerten und unter Normaldruck über 150°C, vorzugsweise über 180°C, siedenden Lösungsmittels durch, wobei das Gemisch auf Temperaturen von 150 bis 300°C, vorzugsweise bis 270°C, erhitzt wird (DE-A 1 059 899). Das Lösungsmittel wird im allgemeinen in der 1- bis 15-fachen Gewichtsmenge, bezogen auf den Polyester, verwendet. Die thermische Spaltung des Polyesters findet vorteilhaft in Gegenwart von 20 %, vorzugsweise von 20 bis 60 % eines sekundären oder tertiären aliphatischen, alicyclischen, 5- oder 6-gliedrigen heterocyclischen Stickstoff und bzw. oder Sauerstoff enthaltenden oder aliphatisch aromatisch substituierten, unter Normaldruck oberhalb 100°C, vorzugsweise oberhalb 150°C, siedenden Amins als Katalysator bei Temperaturen von 160 bis 220°C unter gleichzeitigem Abdestillieren der Sorbinsäure und des Lösungsmittels statt.

Als geeignete Amine seien zum Beispiel genannt: Methyloctadecylamin, Dimethyloctadecylamin, Dimethylhexadecylamin, Dimethyltetradecylamin, Dimethyldodecylamin, Dibutyldodecylamin, N,N',N,N'-Tetramethylhexamethylendiamin, N,N,N'-Trimethyl-N'-phenylethylendiamin, N-Octadecylpyrrolidon, N-Octadecylpiperidin, N-Dodecylmorpholin, N,N'-Dipropylpiperazin, α-Hexylpyrrolidon, Triethylentetramin, Ethyl-bis-(β-ethylamino-ethyl)-amin, 1-Octyldiethylentriamin, Ethylenglycol-bis-(2-methylaminoethyl-ether), Dioctadecylamin, Diethylentriamin, Trioctadecylamin, Trioctylamin, Tricyclohexylamin.

Zur Durchführung der Umsetzung sind als Verdünnungsmittel aliphatische, alicyclische oder aromatische Kohlenwasserstoffe, deren Chlor-, Brom- und Nitroderivate sowie auch Ether und Siliconöle geeignet, deren Siedepunkt bei normalem Druck über 150°C, vorzugsweise über 180°C liegen. Aber auch Ketone, Ester, Carbonsäuren und Alkohole mit dem entsprechenden Siedebereich können als Verdünnungsmittel herangezogen werden, obwohl im allgemeinen die Ergebnisse nicht ganz so gut sind, da sie sich offenbar teilweise mit dem Reaktionsgemisch umsetzen. Es ist zweckmäßig, solche Verdünnungsmittel bzw. Lösungsmittel zu verwenden, die bei normalen Temperaturen flüssig sind, bei normalem Druck unter 300°C, vorzugsweise unter 270°C sieden und mit der Sorbinsäure azeotrope Gemische bilden, so dass sie auch gleichzeitig als Träger oder Schleppmittel dienen, wie Petroleumfraktionen, Dodekan, Tetradekan, 5-Methyldodekan, Dodecen, Dicyclohexylmethan, p-Di-tert.-butylbenzol, 1-Methylnaphthalin, 2-Methylnaphthalin, 1-Ethylnaphthalin, Tetrahydronaphthalin, Diphenyl, Naphthalin, halogenierte aliphatische, cycloaliphatische oder aromatische Kohlenwasserstoffe, wie Dichlordodecan, 1,5-Dibrompentan, Benzotrichlorid, o- und m-Dibrombenzol, Nitroverbindungen, wie Nitrobenzol, 2-Nitrotoluol, Nitrile, wie Benzylcyanid, Carbonylverbindungen, wie Acetophenon oder das heterocyclische 2-Acetylthiophen, heterocyclische Verbindungen, wie Chroman, Thiophen, Ether, wie Resorcindimethylether, Diphenylether, Safrol, Isosafrol, Säuren, wie Önanthsäure, α-Ethylcapronsäure, Caprylsäure, Caprinsäure, Ester, wie Benzoesäureethylester, Phenylessigsäuremethylester und Salicylsäuremethylester (DE-A-1 059 899).

Die folgenden Beispiele erläutern die Erfindung. Als Ausgangsmaterial dient ein polyesterhaltiges Umsetzungsprodukt, das analog der deutschen Auslegeschrift 1 042 573, Beispiel 1, erhalten wird. Dabei werden in ein gerührtes Gemisch aus 800 g Crotonaldehyd, 1200 ml Toluol und 14,2 g Zinkisovalerianat bei einer Temperatur zwischen 25 und 35°C 420 g Keten eingeleitet. Der Überschuß an Crotonaldehyd und das Toluol werden im Vakuum entfernt. Als Rückstand werden 1150 g Polyester in Form einer hochviskosen, braungefärbten Flüssigkeit erhalten. Neben dem Zinkgehalt von 3000 ppm enthält dieses Reaktionsprodukt noch Anteile, die nicht in Hexadiensäuren umgewandelt werden können, wie Diketenpolymerisate und Crotonaldehydharze.

Der in Hexadiensäuren umwandelbare Anteil wurde bestimmt durch basische Verseifung einer Lösung aus 60 g Sorbinsäurepolyester in 120 g Toluol mit 33 g Kaliumhydroxid in 260 g Wasser bei Raumtemperatur. Dabei erhält man in der wäßrigen Phase Kaliumsorbat und das Kaliumsalz der 3-Hydroxy-4-hexensäure, aus denen Hexadiensäuren durch Ansäuern gewonnen werden kann. Durch quantitative Bestimmung der beiden Reaktionsprodukte mittels HPLC kann der in Hexadiensäuren umwandelbare Anteil des Polyesters bestimmt werden. Durch diese milden Bedingungen läßt sich der Polyestergehalt sehr viel genauer als in DE-B 1 282 645 beschrieben, bestimmen. So beträgt der zu Hexadiensäuren umwandelbare Anteil des Rohpolyesters 89 bis 90 % und nicht, wie in DE-B 1 282 645 angenommen, nur 80 %. Die in DE-B-1 282 645 erzielten Ausbeuten müssen daher, siehe Vergleichsbeispiel 1, korrigiert werden.

### Beispiele

### Vergleichsbeispiel 1

In einen Zweihalskolben, der sich in einem Heizbad von 220°C befindet, wird aus einem Tropftrichter ein Gemisch aus 100 g Polyester, 200 g Triethylenglycoldiethylether und 2 g Dimethyloctadecylamin langsam eingetropft. Über einen Destillationsaufsatz werden die Sorbinsäure und das Schleppmittel bei etwa 20 Torr abdestilliert und in der Vorlage verflüssigt. Nach dem Auskristallisieren werden 76 g reine Sorbinsäure erhalten. Das entspricht einer Ausbeute von 76 %, bezogen auf den eingesetzten Rohpolyester. Bezogen auf den Reinpolyester, also unter alleiniger Berücksichtigung des zu Hexadiensäuren spaltbaren Anteils von 90 %, errechnet sich damit eine Ausbeute von 84,4 %.

### Vergleichsbeispiel 2

In einen Zweihalskolben, der sich in einem Heizbad von 220°C befindet, wird aus einem Tropftrichter ein Gemisch aus 100 g Polyester, 200 g Triethylenglycoldiethylether und 3 g Dimethyloctadecylamin langsam eingetropft. Über einen Destillationsaufsatz werden die Sorbinsäure und das Schleppmittel bei etwa 20 Torr abdestilliert und in der Vorlage verflüssigt. Nach dem Auskristallisieren werden 76,3 g reine Sorbinsäure erhalten. Das entspricht einer Ausbeute von 76,3 %, bezogen auf den eingesetzten Rohpolyester. Bezogen auf den Reinpolyester, also unter alleiniger Berücksichtigung des zu Hexadiensäuren spaltbaren Anteils von 90 %, errechnet sich damit eine Ausbeute von 84,7 %.

### Beispiel 3

In einen mit 30 g Dimethyloctadecylamin gefüllten Zweihalskolben, der sich in einem Heizbad von 220°C befindet, wird aus einem Tropftrichter ein Gemisch aus 100 g Polyester, 200 g Triethylenglycoldiethylether und 13 g Dimethyloctadecylamin langsam eingetropft. Über einen Destillationsaufsatz werden die Sorbinsäure und das Schleppmittel bei etwa 20 Torr abdestilliert und in der Vorlage verflüssigt. Nach dem Auskristallisieren werden 83 g reine Sorbinsäure erhalten. Das entspricht einer Ausbeute von 83 % bezogen auf den eingesetzten Rohpolyester. Bezogen auf den Reinpolyester, also unter alleiniger Berücksichtigung des zu Hexadiensäuren spaltbaren Anteils von 90 %, errechnet sich damit eine Ausbeute von 92,8 %.

### Beispiel 4

In einen mit 45 g Dimethyloctadecylamin gefüllten Zweihalskolben, der sich in einem Heizbad von 220°C befindet, wird aus einem Tropftrichter ein Gemisch aus 100 g Polyester und 400 g 2-Ethylhexansäure langsam eingetropft. Über einen Destillationsaufsatz werden die Sorbinsäure und das Schleppmittel bei etwa 20 Torr abdestilliert und in der Vorlage verflüssigt. Nach dem Auskristallisieren werden 84,5 g reine Sorbinsäure erhalten. Das entspricht einer Ausbeute von 84,5 bezogen auf den eingesetzten Rohpolyester. Bezogen auf den Reinpolyester, also unter alleiniger Berücksichtigung des zu Hexadiensäuren spaltbaren Anteils von 90 %, errechnet sich damit eine Ausbeute von 93,9 %.

### Beispiel 5

In einen mit 60 g Dimethyloctadecylamin gefüllten Zweihalskolben, der sich in einem Heizbad von 220°C befindet, wird aus einem Tropftrichter ein Gemisch aus 100 g Polyester, 200 g Triethylenglycoldiethylether und 13 g Dimethyltetradecylamin langsam eingetropft. Über einen Destillationsaufsatz werden die Sorbinsäure und das Schleppmittel bei etwa 20 Torr abdestilliert und in der Vorlage verflüssigt. Nach dem Auskristallisieren werden 86 g reine Sorbinsäure erhalten. Das entspricht einer Ausbeute von 86 % bezogen auf den eingesetzten Rohpolyester. Bezogen auf den Reinpolyester, also unter alleiniger Berücksichtigung des zu Hexadiensäuren spaltbaren Anteils von 90 %, errechnet sich damit eine Ausbeute von 95,5 %.

## Patentansprüche

1. Verfahren zur Herstellung von Sorbinsäure durch thermische Spaltung des aus Crotonaldehyd und Keten hergestellten Polyesters in einem Lösungsmittel, **dadurch gekennzeichnet, dass** man die Spaltung des Polyesters in Gegenwart von 20 bis 100 Gew.-% (bezogen auf den eingesetzten Polyester) eines als Katalysator dienenden
- sekundären oder tertiären aliphatischen oder alicyclischen Stickstoff oder Sauerstoff oder Stickstoff und Sauerstoff enthaltenden Amins oder
- aliphatisch-aromatisch substituierten Amins,
wobei das Amin unter Normaldruck oberhalb 100°C, vorzugsweise oberhalb 150°C, siedet, durchführt.

2. Verfahren nach Anspruch 1, **dadurch gekennzeichnet, dass** man 20 bis 60 Gew.-% (bezogen auf den eingesetzten Polyester) des Amins einsetzt.

3. Verfahren nach Anspruch 1 oder 2, **dadurch gekennzeichnet, dass** als Amin eine oder mehrere der nachfolgenden Verbindungen eingesetzt wird:
Methyloctadecylamin, Dimethyloctadecylamin, Dimethylhexadecylamin, Dimethyltetradecylamin, Dimethyldodecylamin, Dibutyldodecylamin, N,N',N,N'-Tetramethylhexa-methylendiamin, N,N,N'-Trimethyl-N' phenylethylendiamin, N-Octadecylpyrrolidon, N-Octadecylpiperidin, N-Dodecylmorpholin, N,N'-Dipropylpiperazin, a-Hexylpyrrolidon, Triethylentetramin, Ethyl-bis-(β-ethylamino-ethyl)-amin, 1-Octyldiethylentriamin, Ethylenglycol-bis-(2 methylaminoethyl-ether), Dioctadecylamin, Diethylentriamin, Trioctadecylamin,
Trioctylamin und Tricyclohexylamin.

4. Verfahren nach Anspruch 1 bis 3, **dadurch gekennzeichnet, dass** man die Spaltung des Polyesters zur Herstellung der Sorbinsäure bei Temperaturen von 160 bis 220°C unter gleichzeitigem Abdestillieren der Sorbinsäure durchführt.
